# EUROPEAN PATENT APPLICATION

(11) **EP 4 806 450 A1**
(43) Date of publication of application: **16.09.2026**
(21) Application number: 25162675.0
(22) Date of filing: 10.03.2025
(51) Int. Cl.: A61K 38/00, A61K 38/16, A61K 48/00, C07K 7/06, C07K 14/005, C12N 7/00, C12N 15/10, C12N 15/86, C40B 40/02

(54) **NOVEL PEPTIDES WITH SPECIFICITY FOR ENTHOTHELIAL CELLS**

(71) Applicant: Universitätsklinikum Hamburg-Eppendorf, 20246 Hamburg (DE); Universität zu Lübeck, 23538 Lübeck (DE); Universität Augsburg, in Vertretung des Freistaates Bayern, 86159 Augsburg (DE)
(72) Inventor: KÖRBELIN, Jakob, 20246 Hamburg (DE); SCHWANINGER, Markus, 23562 Lübeck (DE); TREPEL, Martin, 86156 Augsburg (DE)
(74) Representative: Eisenführ Speiser

(57) **Abstract**

The invention relates to a peptide, polypeptide, or protein that binds specifically to endothelial cells. The peptide, polypeptide, or protein can be a component of a viral capsid and can be used to target a recombinant viral vector selectively to endothelial tissue after systemic administration to a subject and to ensure expression of one or more transgenes in well-perfused organs, such as the brain, the heart, the kidney, the lung, the liver and the spleen. The invention thus further relates to a recombinant viral vector, preferably an AAV vector, which comprises a capsid containing the peptide, polypeptide, or protein according to the invention and which comprises at least one transgene packaged in the capsid. The viral vector is suitable in particular for the therapeutic treatment of a vascular or metabolic disorder or a vascular or metabolic disease. The invention further relates to cells and pharmaceutical compositions which comprise the viral vector according to the invention.

## Description

The invention relates to a peptide, polypeptide, or protein that binds specifically to endothelial cells. The peptide, polypeptide, or protein can be a component of a viral capsid and can be used to target a recombinant viral vector selectively to endothelial tissue after systemic administration to a subject and to ensure expression of one or more transgenes in well-perfused organs, such as the brain, the heart, the kidney, the lung, the liver and the spleen. The invention thus further relates to a recombinant viral vector, preferably an AAV vector, which comprises a capsid containing the peptide, polypeptide, or protein according to the invention and which comprises at least one transgene packaged in the capsid. The viral vector is suitable in particular for the therapeutic treatment of a vascular or metabolic disorder or a vascular or metabolic disease. The invention further relates to cells and pharmaceutical compositions which comprise the viral vector according to the invention.

### Background of the invention

Gene therapy offers promising therapeutic approaches for a range of diseases that affect a wide variety of organs. The method of gene transfer is of crucial importance for the success of the therapy. Viral gene transfer vectors based on adeno-associated virus (AAV) have proven successful and are already used in several approved gene therapies [1]. The vectors are applied either by intravenous or local injection. While some organs like the liver are transduced rather easily after intravenous injection of different available AAV serotypes, other organs like the brain either require more invasive methods of vector application (like intracerebral injection) or they depend on molecularly tailored AAV capsid variants optimized for the intended target tissue. Multiple attempts have aimed at improving AAV's tropism for different cell types by modifying the viral capsid, including capsid shuffling of AAV serotypes [2-4], attachment or incorporation of cell type-specific antibodies, nanobodies or other ligands like DARPins [5-7], and AAV display peptide libraries [8-11].

However, systemic intravenous application of AAV vectors is currently associated with the problem of insufficient specificity for the target cell populations to be treated. Vascular or metabolic diseases represent a large group of sometimes extremely serious diseases that have not yet been treated with gene therapy due to the absence of viral vectors with the appropriate specificity.

Intravenous application of therapeutic AAV vectors generally appears to be a suitable approach for the treatment of vascular diseases. However, no AAV vectors with sufficiently specific vascular endothelial tropism in humans are currently available. The AAV vectors available to date for use in humans accumulate to a large extend in the liver. For example, AAV vectors used in preclinical and clinical applications have been reported to accumulate in hepatocytes, which can lead to unwanted, sometimes fatal side effects [12, 13].

Engineered AAV capsid libraries have successfully been screened in rodent models to identify AAV mutants with improved targeting properties [14-16]. It has become apparent, though, that AAV mutants which yield excellent results in rodents, regularly do not show the same tropism in primates or humans [17]. It is therefore particularly desirable that the favourable vector tropism occurs in primates and especially in humans.

Several attempts have been made to prepare AAV vector variants that exhibit binding specificity for endothelial cells [18]. EP 2 176 283 B1 describes peptides for targeting AAV vectors to vascular endothelial cells of the central nervous system of mice. Similarly, WO 2023/220386 A1 describes modified AAV capsids comprising amino acid sequences for targeting the viral vectors to the brain microvasculature. Barbon et al. [19] reported the development of a dual hybrid AAV vector for endothelial-targeted expression of von Willebrand factor in mice. Chen et al [20] described an approach for the functional gene delivery to and across brain vasculature of systemically administered AAVs with specificity for brain endothelial cells in rodents.

Thus, the endothelial AAV vectors of the prior art have one or more disadvantages; they either show increased transduction of endothelial cells *in vivo* while still showing strong affinity for hepatocytes, the endothelial tropism is restricted to the endothelium of a single organ and/or the endothelial tropism was shown in rodents and could not be confirmed in mammals. Accordingly, there is a need for a clinically applicable gene therapy system which allows to overcome the disadvantages described hereinabove by providing AAV vectors that efficiently transduce human endothelial cells and, at the same time, accumulate to a lesser extent in the liver than previously known vectors. In particular, it is desirable for numerous applications to provide AAV vectors with high specificity for endothelial cells in general and without a restriction to a specific organ. This would enable the treatment of vascular treatments or vascular disorders that affect several or all organs.

The present invention solves this problem by providing viral vectors for the targeted gene transfer of transgenes into endothelial cells. The viral vectors according to the invention comprise on their capsid surface a previously unknown amino acid sequence that provides for a specific targeting to endothelial cells, including those of well-perfused organs such as brain, lung, heart, kidney, spleen and liver. As such, the viral vectors of the present invention specifically transduce endothelial cells of a patient following systemic administration to the same. The viral vectors described herein show, compared to control vectors, increased targeting of endothelial cells, including the microvascular endothelial cells of well-perfused organs such as the brain, the heart, the lung, the kidney, the spleen and the liver in primates after intravenous application. Accordingly, the viral vectors enable efficient transduction of microvascular endothelial cells at comparatively low transduction of human hepatocytes.

The viral vectors according to the invention also enable a strong and persistent expression of a transgene in the endothelial cells transduced with only minor immune response, and are therefore particularly suitable for gene therapy treatments of certain vascular or metabolic disorders and/or vascular or metabolic diseases. After transduction, the AAV vectors only instigate a minor immune response in the host and are therefore particularly suitable for gene therapy.

### Description of the invention

In the present invention, peptide sequences with specificity for endothelial cells were identified from random AAV2 peptide libraries. As set out in detail in the Examples, AAV2 capsid mutant libraries were prepared and used in multiple selection rounds *in vivo* in the common marmoset, a non-human primate. Particle distribution and enrichment of AAV capsid variants was monitored by next-generation sequencing (NGS) in several organs including brain, heart and kidney. Comprehensive analysis of the NGS data surprisingly revealed the enrichment of several AAV2 capsid mutants which comprised the amino acid sequence DWP (aspartic acid-tryptophan-proline) in well-perfused organs such as the brain, the heart and the kidney. *In vitro* experiments on primary human microvascular endothelial cells isolated from different organs including brain, heart, lung, liver, kidney and spleen confirmed superior transduction of AAV capsid variants displaying the DWP amino acid sequence of endothelial cells compared to natural AAV serotypes 1-9. As such, the amino acid sequence DWP was found to provide endothelial cell binding specificity to peptides, polypeptides and proteins. The present invention therefore provides peptide sequences which are particularly suitable for targeting therapeutic agents such as recombinant viral vectors to endothelial cells of a subject being treated.

A first aspect of the present invention accordingly relates to a peptide, polypeptide, or protein that binds specifically to endothelial cells, wherein the peptide, polypeptide, or protein includes the amino acid sequence DWP. Preferably, the peptide, polypeptide or protein is a recombinant peptide, polypeptide or protein. As used herein, also the term "peptide sequence with specificity for the endothelium" is used to refer to a peptide that includes the amino acid sequence DWP. It is preferred that the peptide, polypeptide, or protein includes the amino acid sequence DWP as part of a hexamer (6mer) peptide. The hexamer peptide has a sequence selected from the group consisting of DWPXXX, XDWPXX, XXDWPX and XXXDWP. Among these, the hexamer peptide sequence DWPXXX is particularly preferred for all aspects and all embodiments of the present invention. In the context of the present disclosure, "X" is construed as any one of the amino acids "A", "R", "N", "D", "C", "Q", "E", "G", "H", "I", "L", "K", "M", "F", "P", "O", "S", "U", "T", "W"*,* "Y", or "V".

Particularly preferably, the peptide, polypeptide, or protein of the invention includes at least one of the amino acid sequences of SEQ ID NO: 1 (DWPATY), SEQ ID NO: 2 (DWPQSM) and SEQ ID NO: 3 (DWPQDL) with the amino acid sequence of SEQ ID NO: 1 (DWPATY) being even further preferred.

The endothelial cells, to which the peptide, polypeptide, or protein of the invention specifically bind, are preferable microvascular endothelial cells. As used herein, the term "microvascular endothelial cells" refers to cells of blood vessels with a diameter of less 100 µm or less.

It is moreover preferred that the peptide, polypeptide, or protein of the invention specifically binds to endothelial cells, preferably microvascular endothelial cells, of at least one and preferably all of the organs of the group consisting of brain, heart, lung, kidney, spleen and liver.

It was surprisingly found that peptides, polypeptides and proteins of the invention bind to endothelial cells in general, meaning that the binding specificity is not restricted to certain organs but instead "panendothelial". This results in a particularly favourable biodistribution of recombinant viral vectors which comprise at least one capsid protein including the amino acid sequence DWP and thus to in numerous possibilities for treating vascular or metabolic disorders and diseases by gene therapy.

In the context of the present disclosure, the term "peptide" refers to a linkage of 2-10 amino acids which are connected to each other by a peptide bond. The term "polypeptide" refers to a linkage of 11-100 amino acids that are connected to each other by a peptide bond. Polypeptides with more than 100 amino acids are referred to herein as a "protein."

In a particularly preferred embodiment of the invention, the amino acid sequence DWP with specificity for endothelial cells that is preferably part of a hexamer peptide which in particular has an amino acid sequence selected from SEQ ID NO: 1 (DWPATY), SEQ ID NO: 2 (DWPQSM) and SEQ ID NO: 3 (DWPQDL), is a part of a capsid protein of a virus. Accordingly, the present invention also pertains to a capsid protein of a viral vector which includes the amino acid sequence DWP that is preferably part of a hexamer peptide that in particular has an amino acid sequence selected from SEQ ID NO: 1 (DWPATY), SEQ ID NO: 2 (DWPQSM) and SEQ ID NO: 3 (DWPQDL). To produce such capsid proteins, a corresponding nucleotide sequence which codes for the peptide with specificity for the endothelium is inserted into the region of the virus genome which codes for a capsid protein of the virus. If the amino acid sequence with specificity for the endothelium is expressed as part of a capsid protein, it can be presented across the surface of the viral vector.

The capsid protein is preferably of an adeno-associated virus (AAV). The AAV can be any of the serotypes described in the prior art, wherein the capsid protein is preferably derived from an AAV of one of the serotypes 2, 4, 6, 8 and 9. A capsid protein of an AAV of serotype 2 is particularly preferred.

The capsid of the AAV wild-type is made up of the capsid proteins VP1, VP2 and VP3, which are overlapping gene products encoded by the cap open reading frame. In its naturally occurring forms, all three proteins have the same C-terminal region. The capsid of AAV comprises in total about 60 copies of the proteins VP1, VP2 and VP3, expressed in a ratio of about 1:1:10. If a nucleotide sequence coding for one of the peptides with specificity for the endothelium described herein is cloned at the C-terminus into the reading frame of VP1 (i.e., in the region that is identical in all three proteins), it can be expected that theoretically 60 of the specific peptides can be found on the capsid surface.

If an AAV vector in the context of the present invention is modified, the nucleotide sequence coding for the peptide with specificity for the endothelium is then preferably inserted into the cap region at the 3' end of the genome. The gene encoding the peptide sequence with specificity for the endothelium can be inserted into the genomic sequence of one of the capsid proteins VP1, VP2 or VP3. The capsid proteins of AAV2 are illustrated by way of example in SEQ ID NO: 4 (VP1), SEQ ID NO: 5 (VP2), and SEQ ID NO: 6 (VP3).

In one particularly preferred embodiment according to the invention, the gene encoding the peptide sequence that binds to endothelial cells is inserted into the reading frame of a VP1 gene, preferably in the VP1 gene of AAV2 encoding the gene product shown in SEQ ID NO: 4 (VP1). It should be noted in this case that the insertion of the cloned sequence does not lead to any change of the reading frame, nor to a premature halt to the translation. The methods required for the above are readily apparent to a person skilled in the art. In a particularly preferred embodiment, the capsid protein has the sequence of SEQ ID NO: 4 (VP1) but comprises an N587Q mutation.

In all three capsid proteins of AVV, sites have been identified at which peptide sequences can be inserted for the homing function [21-26]. Among other things, the arginine which occurs in the VP1 of AAV2 at position 588 (R588) has specifically been proposed for the insertion of a peptide ligand [27-28]. This amino acid position of the viral capsid is apparently involved in the binding of AAV2 to its natural receptor. It has been suggested that R588 is one of four arginine residues which mediates the binding of AAV2 to its natural receptor [29-30]. A modification in this region of the capsid weakens the natural tropism of AAV2, or eliminates it completely.

Accordingly, it is particularly preferred according to the invention that the inventive peptide sequence that binds with specificity to endothelial cells and which includes the amino acid sequence DWP, preferably as part of a hexamer peptide which in particular has an amino acid sequence selected from SEQ ID NO: 1 (DWPATY), SEQ ID NO: 2 (DWPQSM) and SEQ ID NO: 3 (DWPQDL), is present in the region of the amino acids 550-600 of the VP1 protein of AAV2. Accordingly, the amino acid sequence DWP and preferably the hexamer peptide comprising the amino acid sequence DWP which in particular has an amino acid sequence selected from SEQ ID NO: 1 (DWPATY), SEQ ID NO: 2 (DWPQSM) and SEQ ID NO: 3 (DWPQDL) have in particular been inserted in the indicated region by methods that are generally known to the skilled person, such that the obtained protein includes the amino acid sequence DWP, preferably as part of a hexamer peptide which in particular has an amino acid sequence selected from SEQ ID NO: 1 (DWPATY), SEQ ID NO: 2 (DWPQSM) and SEQ ID NO: 3 (DWPQDL), in inserted form in the indicated region of the viral protein used for insertion.

Even more preferably, the peptide sequence that binds with specificity to endothelial cells is present in the region of amino acids 560-600, 570-600, 560-590, 570-590 of the VP1 protein.

Thus, the amino acid sequence DWP that is preferably part of a hexamer peptide comprising the amino acid sequence DWP which in particular has an amino acid sequence selected from SEQ ID NO: 1 (DWPATY), SEQ ID NO: 2 (DWPQSM) and SEQ ID NO: 3 (DWPQDL), adjoin, by way of example directly behind one of the following amino acids of the VP1 protein, which particularly has the sequence of SEQ ID NO: 4: 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599 or 600. It is particularly preferred that the amino acid sequence DWP that is preferably part of a hexamer peptide which in particular has a sequence selected from SEQ ID NO: 1 (DWPATY), SEQ ID NO: 2 (DWPQSM) and SEQ ID NO: 3 (DWPQDL), follows the amino acid residue at position 588 (arginine) of the VP1 protein of SEQ ID NO: 4 (VP1), as shown in the examples. It is possible in this case that one or more, and particularly up to 5 (for example, 1, 2, 3, 4 or 5) flanking amino acids which are the result of the cloning are situated between the arginine residue at position 588 and the first amino acid of amino acid sequence with specificity for the endothelium, preferably at least one of the amino acid sequences of SEQ ID NO: 1 (DWPATY), SEQ ID NO: 2 (DWPQSM) and SEQ ID NO: 3 (DWPQDL). Likewise, one or more, and particularly up to 5 (i.e., 1, 2, 3, 4, or 5) flanking amino acids can be situated behind the last amino acid of the amino acid sequence DWP or the hexamer peptide comprising the amino acid sequence DWP, which is preferably selected from SEQ ID NO: 1 (DWPATY) or SEQ ID NO: 2 (DWPQSM) and SEQ ID NO: 3 (DWPQDL).

The sites and regions in the amino acid sequence of the capsid protein indicated above for VP1 are analogous to the capsid proteins VP2 and VP3 of AAV2. Because the three capsid proteins VP1, VP2 and VP3 differ from AAV2 only by the length of the N-terminal sequence and accordingly have an identical C-terminus, a person skilled in the art will have no problem making a sequence comparison to identify the sites indicated above, for the insertion of the peptide ligands, in the amino acid sequences of VP2 and VP3. As such, the amino acid 588 in VP1 corresponds to position R451 of VP2 (SEQ ID NO: 5) and/or position R386 of VP3 (SEQ ID NO: 6).

Accordingly, in a particular preferred embodiment, the invention pertains to a protein as defined above, wherein the amino acid sequence DWP, or preferably a hexamer peptide comprising the amino acid sequence DWP that in particular has an amino acid sequence selected from SEQ ID NO: 1 (DWPATY), SEQ ID NO: 2 (DWPQSM) and SEQ ID NO: 3 (DWPQDL), is inserted in a capsid protein that has the sequence of SEQ ID NO: 4 (VP1) or an amino acid sequence which is at least 80%, preferably at least 85% and in particular at least 95% identical to full length of the amino acid sequence of SEQ ID NO: 4 (VP1), in particular between the amino acid residues R588 and G589 of the capsid protein.

As described hereinabove, it may be further preferred that the amino acid sequence DWP or preferably the hexamer peptide comprising the amino acid sequence DWP, which preferably has an amino acid sequence selected from SEQ ID NO: 1 (DWPATY), SEQ ID NO: 2 (DWPQSM) and SEQ ID NO: 3 (DWPQDL) comprises at least one of an N-terminal and a C-terminal flanking sequence, each comprising up to 5, in particular up to 3 and particularly preferably 1 amino acid. The N-terminal and the C-terminal flanking sequence preferably consist of G and A residues. Particularly preferably, the N-terminal flanking sequence is G and the C-terminal flanking region is A.

SEQ ID NO: 7 (DWPATY in VP1) shows an example of the sequence of the VP1 protein of AAV2 after introduction of the peptide sequence of SEQ ID NO: 1 (DWPATY). SEQ ID NO: 8 (DWPQSM in VP1) shows an example of the sequence of the VP1 protein of AAV2 after introduction of the peptide sequence of SEQ ID NO: 2 (DWPQSM). SEQ ID NO: 9 (DWPQDL in VP1) shows an example of the sequence of the VP1 protein of AAV2 after introduction of the peptide sequence of SEQ ID NO: 3 (DWPQDL). Due to the cloning, the capsid protein has two additional amino acids which do not occur in the native sequence of the VP1 protein of AAV2. As such, the peptide sequence of SEQ ID NO: 2 (DWPQSM) is flanked at its N-terminus by a glycine in position 589, and at its C- terminus by an alanine in position 597. In addition, the asparagine at position 587 of the native sequence is replaced with a glutamine.

In one particular embodiment, the present invention therefore also relates to a capsid protein which comprises or consists of:
(a) the amino acid sequence of SEQ ID NO: 7 (DWPATY in VP1), SEQ ID NO: 8 (DWPQSM in VP1) or SEQ ID NO: 9 (DWPQDL in VP1);
(b) an amino acid sequence which is at least 80%, preferably at least 90% and particularly preferably at least 95%, identical to the full length of any one of the amino acid sequences of SEQ ID NO: 7 (DWPATY in VP1), SEQ ID NO: 8 (DWPQSM in VP1) and SEQ ID NO: 9 (DWPQDL in VP1); or
(c) a fragment of one of the amino acid sequences defined in (a) or (b).

As used in the present disclosure, the terms "identical" or "percent identity," in the context of two or more nucleic acid or polypeptide sequences, refer to two or more sequences or subsequences that are the same in length and/or have a specified percentage of nucleotides or amino acid residues that are the same, when compared and aligned for maximum correspondence. To determine the percent identity, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = number of identical positions/total number of positions (e.g., overlapping positions) x100). In some embodiments, the two sequences that are compared are the same length after gaps are introduced within the sequences, as appropriate (e.g., excluding additional sequence extending beyond the sequences being compared).

The determination of percent identity or percent similarity between two sequences can be accomplished using a mathematical algorithm. A preferred, non-limiting example of a mathematical algorithm utilized for the comparison of two sequences is the algorithm of Karlin and Altschul, 1990, Proc. Natl. Acad. Sci. USA 87:2264-2268, modified as in Karlin and Altschul,1993, Proc. Natl. Acad. Sci. USA 90:5873-5877. Such an algorithm is incorporated into the NBLAST and XBLAST programs of Altschul et al., 1990, J. Mol. Biol. 215:403-410. BLAST nucleotide searches can be performed with the NBLAST program, score=100, word length=12, to obtain nucleotide sequences homologous to a nucleic acid encoding a protein of interest. BLAST protein searches can be performed with the XBLAST program, score=50, word-length=3, to obtain amino acid sequences homologous to a protein of interest. To obtain gapped alignments for comparison purposes, Gapped BLAST can be utilized as described in Altschul et al., 1997, Nucleic Acids Res. 25:3389-3402. Alternatively, PSI-Blast can be used to perform an iterated search which detects distant relationships between molecules (Id.). When utilizing BLAST, Gapped BLAST, and PSI-Blast programs, the default parameters of the respective programs (e.g., XBLAST and NBLAST) can be used. Another preferred, non-limiting example of a mathematical algorithm utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art and include ADVANCE and ADAM as described in Torellis and Robotti, 1994, Comput. Appl. Biosci. 10:3-5; and FASTA described in Pearson and Lipman,1988, Proc. Natl. Acad. Sci. USA 85:2444-8. Within FASTA, ktup is a control option that sets the sensitivity and speed of the search. If ktup=2, similar regions in the two sequences being compared are found by looking at pairs of aligned residues; if ktup=1, single aligned amino acids are examined. ktup can be set to 2 or 1for protein sequences, or from 1 to 6 for DNA sequences. The default if ktup is not specified is 2 for proteins and 6 for DNA. Alternatively, protein sequence alignment may be carried out using the CLUSTAL W algorithm, as described by Higgins et al., 1996, Methods Enzymol. 266:383-402.

In a further aspect, the invention is directed to a viral capsid, which comprises the peptide, polypeptide or protein according to the invention as described above.

Furthermore, the present invention also provides a nucleic acid encoding a peptide, polypeptide or protein as described above. A nucleic acid which encodes a capsid protein which comprises a peptide, polypeptide or protein according to the invention as described above, is likewise provided. Preferably, the nucleic acid coding for the capsid protein comprises one of the nucleotide sequences of SEQ ID NO: 10 (nucleotide sequence DWPATY in VP1), SEQ ID NO: 11 (nucleotide sequence DWPQSM in VP1) or SEQ ID NO: 12 (nucleotide sequence DWPQDL in VP1) or a nucleotide sequence derived from any of these, with at least 70%, more preferably at least 80%, in particular at least 90% and particularly preferably at least 95% sequence identity to the full length of any one of the nucleotide sequences of SEQ ID NO: 10 (nucleotide sequence DWPATY in VP1), SEQ ID NO: 11 (nucleotide sequence DWPQSM in VP1) and SEQ ID NO: 12 (nucleotide sequence DWPQDL in VP1). A plasmid comprising such a nucleic acid is also provided.

In yet another aspect, the invention relates to a recombinant - *i.e.,* produced by means of genetic engineering techniques - viral vector, having a capsid and at least one transgene packaged therein, wherein the capsid comprises at least one capsid protein including the amino acid sequence DWP, preferably as part of a hexamer peptide which in particular has an amino acid sequence selected from SEQ ID NO: 1 (DWPATY), SEQ ID NO: 2 (DWPQSM) and SEQ ID NO: 3 (DWPQDL). The recombinant viral vector can be a recombinant AAV vector - for example, of serotype 2, 4, 6, 8, 9. AAV vectors of serotype 2 are particularly preferred.

The viral vectors of the present invention show increased targeting of the endothelial cells of well-perfused organs including brain, heart, lung, kidney and spleen in primates after intravenous application compared to control vectors and thus enable extremely efficient transduction of human microvascular endothelial cells from said well-perfused organs with comparatively low transduction of human hepatocytes.

The different AAV serotypes differ mainly by their natural tropism. For example, wild-type AAV2 binds more readily to alveolar cells than to lung epithelial cells, while AAV5, AAV6 and AAV9 are capable of greater transduction of epithelial cells of the respiratory tract. At the nucleic acid level, the various AAV serotypes are highly homologous. For example, serotypes AAV1, AAV2, AAV3 and AAV6 are 82% identical on the nucleic acid level [31]. The capsid of the viral vectors according to the invention comprises one or more transgenes. A gene which has been introduced by genetic engineering into the genome of the vector is termed a transgene. The transgene (or transgenes) can be DNA or RNA. Preferably, it is in the form of a single-stranded DNA (ssDNA) or a double-stranded DNA (dsDNA), such as genomic DNA or cDNA. Preferably, the transgene which is to be transported with the aid of the recombinant viral vector according to the invention is a human gene. Suitable transgenes include, for example, therapeutic genes to replenish a missing gene or to replace a dysfunctional gene in patients. Dysfunctional genes can also be genes which are not expressed in the corresponding target tissue, or are only expressed to an insufficient extent. The present invention is generally not restricted to a specific transgene. Methods known for increasing the packaging capacity of AAV vectors that is in the range of 4.7 kb, such as those described in [32], may be applied to the present invention.

In one preferred embodiment, the transgene which is expressed from the viral vector according to the invention is a gene encoding for a protein selected from the group consisting of lyosomal enzymes, transport proteins, signalling proteins, ectonucleotidases and proteins associated to pulmonary arterial hypertension (PAH). Preferred lysosomal enzymes are hexosaminidase A (HEXA), hexosaminidase B (HEXB) and Niemann-Pick intracellular cholesterol transporter 2 (NPC2). A preferred transport protein is monocarboxylate transporter 8 (MCT8, encoded by the *SLC16A2* gene). A preferred signalling protein is the inhibitor of nuclear factor kappa-B kinase subunit gamma (IKBKG also known as NEMO). Preferred ectonucleotidases are ectonucleoside triphosphate diphosphohydrolase-1 (ENTPD1, also known as CD39) and 5'-nucleotidase (5'NT, also known as CD73). Preferred proteins associated to PAH are nitric oxide synthase (NOS), bone morphogenic protein receptor 2 (BMPR2) and FOXF1 transcription factor. NOS is in particular endothelial NOS (eNOS) or inducible NOS (iNOS).

The viral vectors of the present invention are particularly suitable for use in a method for the treatment of a vascular disorder or a vascular disease as well as for the treatment of a metabolic disorder or a metabolic disease. Vascular disorders and diseases in the context of the invention include, in particular, arteriosclerosis, pulmonary arterial hypertension, in-continentia pigmenti, Allan-Herndon-Dudley syndrome, GLUT1 deficiency syndrome, diabetic small vessel diseases, vasculitis and renal vascular diseases. In one particularly preferred embodiment, the viral vectors of the present invention are used for the treatment of pulmonary arterial hypertension. Metabolic disorders and diseases in the context of the invention are preferably lysosomal storage disorders and diseases and are in particular selected from the group consisting of globoid cell leukodystrophy, neuronal ceroid lipofuscinosis, Niemann-Pick disease type C1, Niemann-Pick disease type C2, mucopoly-saccharidosis, CLN8 Batten disease, metachromatic leukodystrophy, Krabbe disease, Gaucher disease, Sandhoff disease, Tay-Sachs disease, Fabry disease, Pompe disease, and cystinosis.

The vectors can also be used to encode or transport antisense-RNA, ribozymes, or the like into the endothelial target tissue. It is also preferred that the viral vector comprises a gene encoding siRNA or shRNA, in particular against HIF2alpha that can be used to treat pulmonary arterial hypertension. Furthermore, vectors according to the invention can also contain transgenes encoding secretory proteins that are intended for systemic administration in the bloodstream. Such secretory proteins can be efficiently deposited in the bloodstream via the cardiovascular system.

As used herein, the term "subject" indicates any human or animal organism that can be infected by AAV vectors. Preferably, the subject being treated is a mammal such as a human, a primate, a mouse or a rat. In one preferred embodiment, the subject to be treated is a human. After transfection into the subject, the vector brings about a site-specific expression of the transgene in the endothelial cells.

The transgene can be present in the viral vector in the form of an expression cassette, which in addition to the sequence of the transgene to be expressed comprises further elements necessary for expression, such as a suitable promoter which controls the expression of the transgene after infection of the appropriate cells. Suitable promoters include, in addition to the AAV promoters, for example the cytomegalovirus (CMV) immediate early promoter, the chicken beta actin/cytomegalovirus hybrid promoter (CAG), endothelial cell-specific promoters, as well as steroid promoters and metallothionein promoters. Endothelial cell-specific promoters are preferred, in particular the VE cadherin promoter and the occluding promoter. As used herein, an endothelial cell-specific promoter is a promoter whose activity in endothelial cells is at least 2-fold, 5-fold, 10-fold, 20-fold, 50-fold or 100-fold higher than in a cell which is not an endothelial cell. Preferably, this promoter is a human promoter. The expression cassette can also include an enhancer element for increasing the expression levels of exogenous protein to be expressed. Furthermore, the expression cassette can include polyadenylation sequences, such as the SV40 polyadenylation sequences or polyadenylation sequences of bovine growth hormone.

The viral vectors according to the invention can, preferably as part of one of their capsid proteins, include the amino acid sequence DWP, preferably as part of a hexamer peptide which more preferably has the amino acid sequence DWPXXX and particularly preferably has an amino acid sequence selected from SEQ ID NO: 1 (DWPATY), SEQ ID NO: 2 (DWPQSM) and SEQ ID NO: 3 (DWPQDL).

Viral vectors with capsids which comprise one of the peptide sequences according to the invention bind specifically to endothelial cells. As used throughout the present disclosure, a "specific" binding of the vectors according to the invention means that the vectors accumulate after systemic administration in particularly large proportions on or in the endothelial cells. This means that more than 40% of the originally administered vector genomes accumulate in the endothelial cells, or in the area of the endothelial cells, while less than 60% accumulate in other cells or tissues, or the area thereof. It is preferred that more than 50%, 60%, 70%, 80%, 90%, 95%, or even more than 99% of the originally administered vector genomes accumulate in, on, or in direct proximity of the endothelial cells. Specific transduction of the vectors can also be determined via the expression of the transgene, in particular when non-specific promoters are used. In the case of viral vectors that specifically transduce endothelial cells, more than 50% of the total expression of the transgene occurs in, on, or in direct proximity of the endothelial cells, while less than 50% of the expression can be observed in, or in the region of, other tissues. It is preferred, however, that more than 60%, 70%, 80%, 90%, 95%, or even more than 99% of the total measured expression of the transgene occurs in, on, or in direct proximity of, the endothelial cells.

A person skilled in the art will easily be able to determine the specific binding of the vectors according to the invention to endothelial cells of the lung and the expression of the transgene introduced using the vectors. Methods for measuring the specificity of transduction and expression, suitable for this purpose, are shown in the examples below.

Preferably, the binding activity of the vectors according to the invention, as can be determined by distribution of the vector genomes or expression of the transgene, for endothelial cells is at least 2-fold, 5-fold, 10-fold, 20-fold, 50-fold, 75-fold, 100-fold, 150-fold, 200-fold, 250-fold, 500-fold, 600-fold, 700-fold, 800-fold, 900-fold, 1000-fold, 2000 fold, 5000- fold, or 10,000-fold higher than the binding activity for a control cell which is not a lung endothelium cell (such as a spleen cell or a hepatocyte).

The invention also relates to a cell that contains a peptide, polypeptide or protein according to the invention, a nucleic acid encoding the same, a plasmid comprising such a nucleic acid, or a recombinant AAV vector as described above. It is preferably a human cell or cell line. The cell preferably present the peptide, polypeptide or protein according to the invention on its surface, in particular as a part of a membrane-associated or transmembrane protein.

In one embodiment, a cell has been, for example, obtained from a human subject by biopsy and then transduced with the viral vector in an ex vivo procedure. The cell can then be re-implanted in the subject, or be supplied in other ways to the subject - for example by transplantation or infusion. The likelihood of rejection of transplanted cells is lower when the subject from which the cell was derived is genetically similar to the subject to which the cell is administered. Preferably, therefore, the subject to whom the transfected cells are supplied is the same subject from which the cells were previously obtained. The cell is preferably an endothelial cell, particularly a cell of the human pulmonary endothelium. The cell to be transduced can also be a stem cell, such as a human adult stem cell. It is particularly preferred according to the invention that the cells to be transfected are autologous cells that have been transfected ex vivo with the viral vector according to the invention, for example the recombinant AAV2 vector described above. The cells are preferably used in a method for treating a vascular or metabolic disorder or a vascular or metabolic disease in a subject.

In another aspect, the invention relates to a method for producing an AAV vector in which a plasmid is used which encodes a capsid protein, the same including the amino acid sequence DWP, preferably as part of a hexamer peptide which in particular has an amino acid sequence of SEQ ID NO: 1 (DWPATY) or SEQ ID NO: 2 (DWPQSM) and SEQ ID NO: 3 (DWPQDL). The basic method of producing recombinant AAV vectors comprising a transgene to be expressed is described in the prior art sufficiently [33]. HEK 293-T cells are transfected with three plasmids. A first plasmid contains the cap and rep regions of the AAV genome, but the naturally occurring inverted repeats (ITRs) are missing. The cap region of this plasmid contains a gene encoding at least one modified capsid protein, i.e., a gene which comprises the peptide sequence according to the invention. A second plasmid comprises a transgene expression cassette which is flanked by the corresponding ITRs, which constitute the packaging signal. The expression cassette is therefore packaged into the capsid in the course of the assembly of the viral particles. The third plasmid is an adenoviral helper plasmid, on which are encoded the helper proteins E1A, E1B, E2A, E4orf6, VA, which are required for AAV replication in the HEK 293-T cells.

Conditions which allow the accumulation and purification of the recombinant vectors according to the invention are known in the art. The vectors according to the invention can be purified, for example, by gel filtration processes - for example using cross-linked beaded agarose (e.g. Sepharose^{®}, Cytiva, USA) - desalinated, and subsequently purified by filtration. Other purification methods can further comprise a cesium chloride or iodixanol gradient ultracentrifugation process. Purification reduces potentially detrimental effects in the subject to which the adeno-associated viral vectors are administered. The administered virus is substantially free of wild-type replication-competent virus. The purity of the virus can be checked by suitable methods such as PCR amplification.

In a further aspect, the invention provides a pharmaceutical composition which contains at least one of a peptide, polypeptide, or protein, a viral capsid, a nucleic acid, a plasmid, or a recombinant viral vector as defined herein above. Preferably, the pharmaceutical composition contains a recombinant viral vector of the present invention, particularly an AAV vector. The viral vector in this case is administered in a therapeutically effective amount to the patient, i.e., in an amount sufficient to considerably improve at least one symptom of the vascular disorder or vascular disease being treated, or to prevent the progression of the disease. Symptoms that are regularly associated with a vascular disorder or vascular disease include epileptic seizures, paresis, paralysis, intellectual disability, dementia, motor symptoms, blindness, visual problems, memory impairment, gait disturbance, ataxia, fatigue, shortness of breath, edema, proteinuria and abnormal blood pressure. A therapeutically effective amount of the vector according to the invention causes a positive change in one of the mentioned symptoms, *i.e.,* a change which results in the phenotype of the affected subject approximating the phenotype of a healthy subject who does not suffer from a vascular pulmonary disorder or disease.

In one preferred embodiment according to the invention, the administration of the viral vector occurs in an amount which leads to a complete or substantially complete healing of the vascular disorder or disease. The pharmaceutical composition accordingly comprises a therapeutically effective dose of the vector according to the invention. A therapeutically effective dose will generally be non-toxic for the subject who undergoes the treatment.

The exact amount of viral vector which must be administered to achieve a therapeutic effect depends on several parameters. Factors that are relevant to the amount of viral vector to be administered are, for example, the route of administration of the viral vector, the nature and severity of the lung disease, the disease history of the patient being treated, and the age, weight, height, and health of the patient to be treated. Furthermore, the expression level of the transgene which is required to achieve a therapeutic effect, the immune response of the patient, as well as the stability of the gene product are relevant for the amount to be administered. A therapeutically effective amount of the viral vector can be determined by a person skilled in the art on the basis of general knowledge and the present disclosure.

The viral vector is preferably administered in an amount corresponding to a dose of virus in the range of 1.0 x 10¹⁰ to 1.0 x 10¹⁴ vg/kg (virus genomes per kg body weight), although a range of 1.0 x 10¹² to 1.0 x 10¹⁴ vg/kg is more preferred, and a range of 5.0 x 10¹² to 5.0 x 10¹³ is still more preferred. A virus dose of approximately 1 x 10¹³ vg/kg is most preferred. The amount of the viral vector to be administered, such as the AAV2 vector according to the invention, for example, can be adjusted according to the strength of the expression of one or more transgenes.

The viral vector of the present invention, such as the preferred AVV2 vector according to the invention, for example, can be formulated for various routes of administration - for example, for oral administration as a capsule, a liquid or the like. However, it is preferred that the viral vector is administered parenterally, preferably by intravenous injection or intravenous infusion. Compositions which are suitable for administration by injection and/or infusion typically include solutions and dispersions, and powders from which corresponding solutions and dispersions can be prepared. Such compositions will contain the viral vector and at least one suitable pharmaceutically acceptable carrier. Suitable pharmaceutically acceptable carriers for intravenous administration include bacteriostatic water, Ringer's solution, physiological saline, phosphate buffered saline (PBS) and Cremophor EL^{™}. Sterile compositions for the injection and/or infusion can be prepared by introducing the viral vector in the required amount into an appropriate carrier, and then sterilizing by filtration. Compositions for administration by injection or infusion should remain stable under storage conditions after their preparation over an extended period of time. The compositions can contain a preservative for this purpose. Suitable preservatives include chlorobutanol, phenol, ascorbic acid and thimerosal. The preparation of corresponding formulations and suitable adjuvants is described, for example, in "Remington: The Science and Practice of Pharmacy," Lippincott Williams & Wilkins; 21st edition (2005).

In a further aspect, the invention relates to a method for the therapeutic treatment of a vascular or metabolic disorder or a vascular or metabolic disease, wherein at least one of a peptide, polypeptide, or protein, a viral capsid, a nucleic acid, a plasmid, or a recombinant viral vector as defined herein above, is administered to a subject. Preferably, the method comprises that a recombinant viral vector according to the invention, preferably an AAV vector as described above, is administered to a subject. The recombinant vector comprises a capsid which has at least one capsid protein that includes the amino acid sequence DWP, preferably as part of a hexamer peptide which in particular has an amino acid sequence selected from SEQ ID NO: 1 (DWPATY), SEQ ID NO: 2 (DWPQSM) and SEQ ID NO: 3 (DWPQDL). The viral vector further comprises a transgene, for example a therapeutic gene, which is useful for the treatment of the vascular or metabolic disorder or the vascular or metabolic disease. After administration to the subject being treated, preferably by systemic administration such as intravenous injection or infusion, for example, the vector brings about the specific expression of the gene in the endothelial cells of the lung or in other organs.

In yet another aspect, the invention is directed to the use of a peptide, polypeptide, or protein, a viral capsid, a nucleic acid, a plasmid, or a recombinant viral vector as defined herein above for the treatment of a vascular or metabolic disorder or a vascular or metabolic disease in a subject. The use of the recombinant viral vector of the present invention, preferably an AAV vector as described above, for the treatment of a vascular or metabolic disorder or a vascular or metabolic disease in a subject is preferred. The recombinant vector comprises a capsid which has at least one capsid protein that includes the amino acid sequence DWP, preferably as part of a hexamer peptide which in particular has an amino acid sequence selected from SEQ ID NO: 1 (DWPATY), SEQ ID NO: 2 (DWPQSM) and SEQ ID NO: 3 (DWPQDL). The viral vector further comprises a transgene, for example a therapeutic gene, which is useful for the treatment of the vascular or metabolic disorder or a vascular or metabolic disease. After administration to the subject being treated, preferably by systemic administration such as intravenous injection or infusion, for example, the vector brings about the specific expression of the gene in the endothelial cells of the lung.

### BRIEF DESCRIPTION OF THE FIGURES

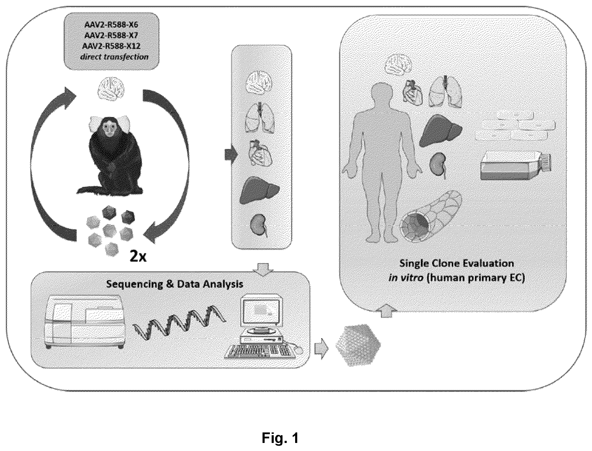

**Figure 1** shows a schematic overview of the selection process. Random AAV2 peptide libraries, which present random peptides of 6, 7 or 12 amino acids in length in their receptor binding domain (inserted after amino acid R588 in the AAV2 VP1 protein), were selected over two selection rounds in common marmosets (Callithrix jacchus) with the brain as the predefined target organ. Then, the distribution of the AAV variants in the brain, lung, heart liver and kidney was examined and evaluated using bioinformatics. The most promising capsid variants were used to produce luciferase vectors and gene expression was analysed in primary human microvascular endothelial cells from the aforementioned organs.

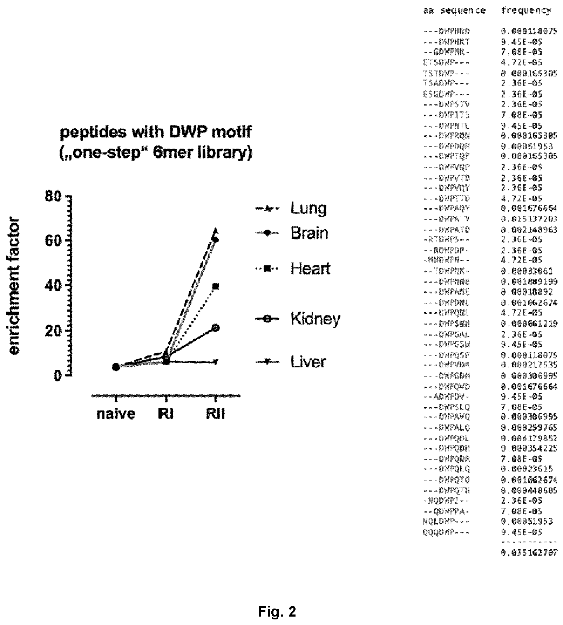

**Figure 2** shows the enrichment of the peptides comprising the amino acid sequence DWP during the selection process. After each round of selection, the AAV library particles recovered from the brain and peripheral organs were analyzed by next-generation sequencing to monitor amino acid distribution and potential peptide motifs. Left: change in the frequency of the DWP motif in peptides of the AAV2-6mer peptide library during the course of selection. Right: isolated AAV-presented peptides with a DWP motif from the brain of common marmosets. The frequency of the individual DWP-bearing peptides is indicated.

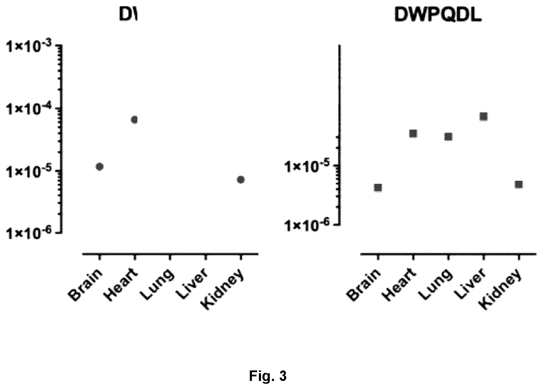

**Figure 3** shows the biodistribution of individual AAV mutants comprising the peptide sequences DWPATY and DWPQDL after two selection rounds in various organs in the common marmoset monkey (n= 1 animal).

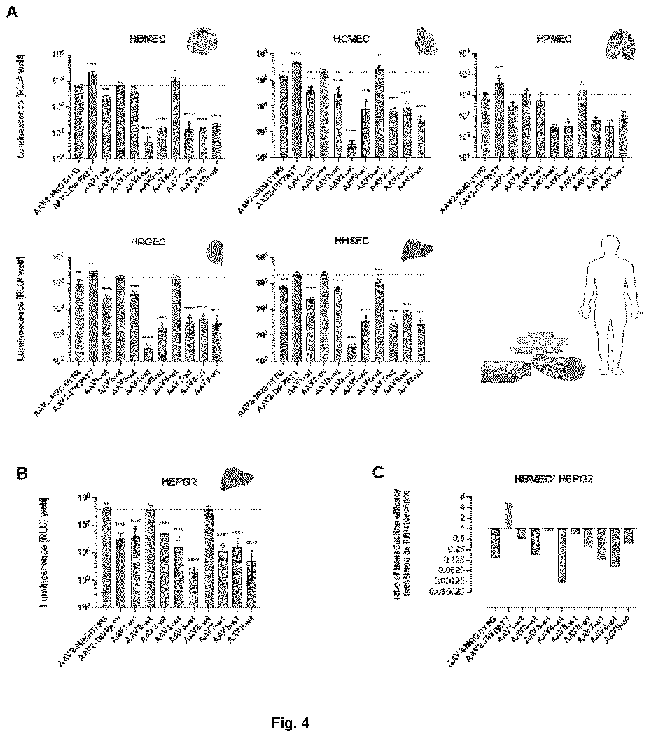

**Figure 4** shows the vector-mediated transgene expression in primary human endothelial cells from various organs. The transduction efficiency of the selected AAV capsid variant AAV2-DWPATY (second bar in each panel) and the control AAV2-MRGDTPG (first bar in each panel) and other AAV serotype controls (AAV 1-9) was evaluated using primary human endothelial cells from various organs. The human primary cells (passages 1-5) were incubated with luciferase reporter vectors at a multiplicity of infection (MOI) of 25,000 vector copies per cell. Vector-mediated bioluminescence was measured after 48 h. Bar graphs represent mean +/- SD with individual data points indicated (n = 5 individual experiments, each in technical triplicates). Transduction efficiency was compared to wild-type AAV2, indicated as dashed line. Statistical analysis was performed by one-way ANOVA followed by Dunnett's post-test. * = p < 0.05; ** = p < 0.01; *** = p < 0.001; **** = p < 0.0001. A) Vector-mediated transgene expression in human brain microvascular endothelial cells (HBMEC), human cardiac microvascular endothelial cells (HCMEC), human pulmonary microvascular endothelial cells (HPMEC), human renal glomerular endothelial cells (HRGEC) and human sinusoidal endothelial cells of the liver (HHSEC). B) Vector-mediated transgene expression in the human hepatoma cell line HEPG2. C) Ratio of transgene expression in HCMEC & HEPG2 as an indicator of endothelial cell (EC) selectivity.

### EXAMPLES

The invention will be illustrated by the following non-limiting examples. Power analysis and sample size estimations were performed using the G*power 3.1.9.7 software (Heinrich Heine Universität Düsseldorf). Statistical analyses of vector distribution and gene expression experiments were performed using the Prism 9 Software (Graphpad). After analyzing data for normal distribution by the Kruskal Wallis test, one-way ANOVA and Dunnett's multiple comparison were performed to test for differences in vector distribution or gene expression.

### Example 1: Peptide identification using random AAV display peptide libraries

Random AAV display peptide libraries were prepared to identify AAV capsid variants with binding specificity for endothelial cells. These libraries are mixtures of up to about one hundred million (100,000,000) different AAV capsids, each carrying a unique, randomly assembled amino acid sequence peptide on its surface that can mediate binding to and internalization into certain target cells and/or organs.

The random AAV peptide libraries were prepared as described in US 20070172460 A1 and [9, 34]. Then, multiple rounds of *in vivo* selections on brain tissue in living common marmosets were carried out according to the method described in [35].

### Example 2: Next-generation sequencing (NGS) and NGS data analysis

After the first and after the second selection round AAV library DNA was isolated and PCR-amplified with AAV cap-specific primers from marmoset brain and control tissues.

NGS sequencing and analysis of the obtained data was carried out as previously described [34].

Comprehensive analysis of the NGS data surprisingly revealed that variants comprising the amino acid sequence "DWP" enrich during the screening process of Example 1. About 3.5% of all hexamer library particles recovered from the brain of the second selection round accounted for DPW-harboring clones (Fig. 2, right panel). During the screening, the DWP motif strongly enriched in different analyzed organs with exception of the liver (Fig. 2, left panel). In the brain and the lung, the DWP motif was most prominently enriched being 60-fold more abundant than expected (expected frequency = 0.000583 considering random amino acid distribution). Slightly less profound enrichment of the DWP-motif was seen in the heart (40-fold) and the kidney (21-fold) (Fig. 2, left panel).

### Example 3: Biodistribution analysis in marmoset monkeys

The biodistribution of the most promising individual AAV variants and some of the most frequent but potentially non-specific controls during the screening procedure in marmoset monkeys was analyzed, as shown in Fig. 3. Increased homing to brain (2.5-fold), heart (10-fold) and lung (6-fold) for the DWP-presenting clones DWPATY and DWPQDL in comparison to control variants displaying commonly observed amino acids N, V, T and R in the first peptide positions (NETRTVQ and NNVRGED) was found.

### Example 4: Production and quantification of recombinant AAV2 vectors

ITR-less AAV1-9 packaging plasmids each harboring the AAV2 *rep* gene and the cap gene of one the respective individual AAV serotypes as well as the capsid-modified AAV2 plasmid pXX2-187 harboring the peptide insertions site were used to transfect HEK 293T cells in combination with CAG promoter-driven GFP or luciferase reporter transfer plasmids as well as the pXX6 adenoviral helper plasmid using Polyfect transfection reagent (Qiagen) according to the manufacturer's instructions. 72h after transfection, particles from cell culture supernatant were precipitated by 10 g PEG8000 and 5.8 g NaCl per 100 ml supernatant, and purified by iodixanol density gradient ultracentrifugation together with the AAV particles of lysed cells after three freeze-thaw cycles. Therefore, a discontinuous iodixanol gradient was prepared by subsequently underlying the harvested library particles with 15, 25, 40, and 54% iodixanol solutions, followed by ultracentrifugation at 230,000 g for 70 min. The purified library particles were aspirated from the layer containing 40% iodixanol and dialyzed against HBSS. The virus titer was determined from 1:10,000 diluted samples at genomic level by real-time PCR (see above).

### Example 5: Analysis of AAV biodistribution

AAV library copy numbers were determined by qPCR in extracted total tissue DNA from the different organs with Cap-specific primers (see above) using approx. 40 ng total tissue

DNA as template. To analyze the biodistribution of individual AAV variants, their relative frequencies in the different organs (determined by NGS) was multiplied with the amount of AAV library genomes recovered from these organs (determined by qPCR). AAV copy numbers were normalized to the amount of injected particles per kg bodyweight and are indicated as percentage of injected particles/kg bodyweight recovered from 1 µg total tissue DNA. Since the sequencing depth did not cover the diversity of the naive AAV library injected into the first animals, the first screening rounds were not included in the biodistribution analysis of individual AAV variants (resulting in n= 3 and n = 1 analyzed animal for the first and second screening campaign, respectively).

### Example 6: Assessment of AAV luciferase reporter expression in human primary EC

Primary human endothelial cells from different organs were purchased from Science Cell and grown in the recommended medium by the same supplier at 37°C and 5% CO₂: brain microvascular EC (HBMEC #), heart microvascular EC (HCMEC #), lung microvascular EC (HPMEC #),hepatic sinusoidal EC (HHSEC #). Human hepatoma cells (HEP-G2) were purchased from the German collection of Microorganisms and Cell Cultures GmbH (DSMZ) and grown in RPMI medium (Gibco) and 10% FCS. To assess the transduction efficacy of different AAV capsids (AAVI-9 as well as AAV2 capsid mutants) on primary human EC, we infected cells of early passage (1-5) with luciferase reporter vectors at a MOI of 25,000 genomic vector copies per cell in a minimal amount of serum-free-medium. Two hours after virus addition, the wells were filled up with full medium containing the recommended amount of FCS. Transgene expression was monitored after 72 h using Luciferase Assay Reagent (Promega #E1483) and a luminescence plate reader (Tecan). Five individual experiments, each in technical triplicates, were performed per cell type.

As shown in Fig. 4, it was found that the analyzed DWP-harboring AAV2-DWPATY variant showed an at least doubled transduction efficacy on human brain microvascular EC (HBMEC; 2.75-fold), human cardiac microvascular EC (HCMEC; 2.3-fold) and human pulmonary microvascular EC (HPMEC; 3.3-fold) compared to wildtype AAV2. No relevant difference in the transduction efficacy between wildtype AAV2 and AAV2-DWPATY was seen on human renal glomerular EC (HRGEC) and human hepatic sinusoidal EC (HHSEC) as shown in Figure 4A, which is consistent with the biodistribution in marmoset monkeys as shown in Fig. 2. Opposed to vascular EC, human hepatoma cells (HEPG2) showed over 90% reduced transduction by the AAV2-DWPATV variant compared to wildtype AAV2 (Fig. 4B). The AAV2-DWPATY variant was found to transducing human brain microvascular EC more efficiently than HEPG2 human hepatoma cells, making the AAV2-DWPATY variant over 30-times more specific for HBMEC than the parental wildtype AAV2 (Fig. 4C; 5.6-fold vs. 0.18-fold expression ratio HBMEC/ HEPG2).

### REFERENCES

[1] Pupo, A., et al., Mol Ther, 2022. 30(12): p. 3515-3541.
[2] Li, W. et al., Mol Ther 16, 1252-1260, doi:10.1038/mt.2008.100 (2008).
[3] Koerber, J. T. et al., Mol Ther 16, 1703-1709, doi:10.1038/mt.2008.167 (2008).
[4] Herrmann, A. K. et al., ACS Synth Biol 8, 194-206, doi:10.1021/acssyn-bio.8b00373 (2019).
[5] Kuklik, J. et al., Int J Mol Sci 22, doi:10.3390/ijms22158355 (2021).
[6] Eichhoff, A. M. et al., Mol Ther Methods Clin Dev 15, 211-220, doi:10.1016/j.omtm.2019.09.003 (2019).
[7] Hartmann, J. et al., Mol Ther Methods Clin Dev 10, 128-143, doi:10.1016/j.omtm.2018:07.001 (2018).
[8] Perabo, L. et al., Mol Ther 8, 151-157, doi:10.1016/s1525-0016(03)00123-0 (2003).
[9] Muller, O. J. et al., Nat Biotechnol 21, 1040-1046, doi:10.1038/nbt856 (2003).
[10] Varadi, K. et al.,. Gene Ther 19, 800-809, doi:10.1038/gt.2011.143 (2012).
[11] Wang, Y. et al., Mol Ther Nucleic Acids 28, 293-306, doi:10.1016/j.omtn.2022.03.017 (2022).
[12] High-dose AAV gene therapy deaths. Nat Biotechnol, 2020. 38(8): p. 910.
[13] Venditti, C.P., Nat Biotechnol, 2021. 39(1): p. 24-26.
[14] Deverman, B. E. et al., Nat Biotechnol34, 204-209, doi:10.1038/nbt.3440 (2016).
[15] Korbelin, J. et al., EMBO Mol Med 8, 609-625, doi:10.15252/emmm.201506078 (2016).
[16] Korbelin, J. et al., Mol Ther 24,1050-1061, doi:10.1038/mt.2016.62 (2016).
[17] Liguore, W. A. et al., Mol Ther 27, 2018-2037, doi: 10.1016/j.ymthe.2019.07.017 (2019).
[18] Hennigs, J.K. et al., Cells, 2021. 10(10).
[19] Barbon E et al., Gene Ther. 2023 Apr;30(3-4):245-254. doi: 10.1038/s41434-020-00218-6.
[20] Chen, X. et al., Nat Commun 14, 3345 (2023). https://doi.org/10.1038/s41467-023-38582-7
[21] Shi and Bartlett, 2003, Mol Ther, 7: 515-525
[22] Loiler et al., 2003, Gene Ther, 10: 1551-1558
[23] Rabinowitz et al., 1999, Virology, 265: 274-285
[24] Wu et al., 2000, J Virol, 74: 8635-8647
[25] Shi et al., 2001, Hum Gene Ther, 12: 1697-1711
[26] Warrington et al. 2004, J Virol, 78: 6595-6609
[27] Girod et al. 1999, Nat Med, 5: 1052-1056
[28] Grifman et al. 2001, Mol Ther, 3: 964-975
[29] Opie et al., 2003, J Virol, 77: 6995-7006
[30] Kern et al., 2003, J Virol, 77: 11072-11081
[31] Russell et al., 1998, J Virol, 72: 309-319
[32] Marrone, L. et al., 2022, Expert Opinion on Biological Therapy, 22(9), 1163-1176.
[33] Xiao et al., 1998, J Virol, 72: 2224-2232
[34] Korbelin, J., et al., Gene Ther, 2017. 24(8): p. 470-481.
[35] Korbelin, J. and M. Trepel, Hum Gene Ther Methods, 2017. 28(3): p. 109-123.

## Claims

1. A peptide, polypeptide, or protein that binds with specificity to endothelial cells, **characterized in that** it includes the amino acid sequence DWP, preferably as part of a hexamer peptide which in particular has an amino acid sequence selected from SEQ ID NO: 1, SEQ ID NO: 2 and SEQ ID NO: 3.

2. The protein according to claim 1, the same being a capsid protein of a viral vector which preferably is an adeno-associated virus (AAV), in particular of an AAV of a serotype selected from the group consisting of serotypes 2, 4, 6, 8, and 9 and particularly preferably an AAV of serotype 2.

3. The protein according to claim 2, wherein the amino acid sequence DWP that is preferably part of the hexamer peptide which in particular has an amino acid sequence selected from SEQ ID NO: 1, SEQ ID NO: 2 and SEQ ID NO: 3 is present in the region of amino acids 550-600 of the capsid protein.

4. The protein according to one of the claims 1 - 3, comprising the following:
(a) the amino acid sequence of SEQ ID NO: 7, SEQ ID NO: 8 or SEQ ID NO: 9;
(b) an amino acid sequence which is at least 80%, preferably at least 90% and particularly preferably at least 95%, identical to the full length of any one of the amino acid sequences of SEQ ID NO: 7, SEQ ID NO: 8 and SEQ ID NO: 9; or
(c) a fragment of one of the amino acid sequences defined in (a) or (b).

5. A viral capsid which has a peptide, polypeptide, or protein according to one of the claims 1 - 4.

6. A nucleic acid which encodes a peptide, polypeptide, or protein according to one of the claims 1 - 4.

7. A plasmid which has a nucleic acid according to claim 6.

8. A recombinant viral vector, having a capsid and a transgene packaged therein, wherein the capsid comprises at least one capsid protein having the amino acid sequence DWP, preferably as part of a hexamer peptide which in particular has an amino acid sequence selected from SEQ ID NO: 1, SEQ ID NO: 2 and SEQ ID NO: 3.

9. The recombinant viral vector according to claim 8, the same being a recombinant AAV vector, preferably an AAV vector of a serotype selected from the group including serotypes 2, 4, 6, 8, and 9 and in particular an AAV vector of serotype 2.

10. The recombinant AAV vector according to claim 8 or 9, wherein the transgene, which is preferably in the form of an ssDNA or a dsDNA, encodes a protein selected from the group consisting of of lyosomal enzymes, transport proteins, signalling proteins, ectonucleotidases and proteins associated to pulmonary arterial hypertension (PAH).

11. The recombinant AAV vector according to claim 9 or 10, for use in a method for the treatment of a vascular or metabolic disorder or a vascular or metabolic disease in a subject, the vascular disorder or the vascular disease being preferably selected from the group consisting of arteriosclerosis, pulmonary arterial hypertension, incontinentia pigmenti, Allan-Herndon-Dudley syndrome, GLUT1 deficiency syndrome, diabetic small vessel diseases, vasculitis and renal vascular diseases, and wherein the subject is preferably a mammal, in particular a human.

12. The recombinant AAV vector for use in a method according claim 11, wherein the vector is formulated for intravenous administration.

13. A cell which contains a peptide, polypeptide, or protein according to one of the claims 1 - 4, a viral capsid according to claim 5, a nucleic acid according to claim 6, a plasmid according to claim 7, or a recombinant viral vector according to one of the claims 8-10.

14. A pharmaceutical composition which contains a peptide, polypeptide, or protein according to one of the claims 1 - 4, a viral capsid according to claim 5, a nucleic acid according to claim 6, a plasmid according to claim 7, or a recombinant viral vector according to one of the claims 8 - 10.

15. Use of a peptide, polypeptide, or protein according to one of the claims 1 - 4, a viral capsid according to claim 5, a nucleic acid according to claim 6, a plasmid according to claim 7, or a recombinant viral vector according to one of the claims 8 - 10 for the treatment of a vascular or metabolic disorder or a vascular or metabolic disease in a subject.
